# EUROPEAN PATENT APPLICATION

(11) **EP 4 169 928 A1**
(43) Date of publication of application: **26.04.2023**
(21) Application number: 21204527.2
(22) Date of filing: 25.10.2021
(51) Int. Cl.: C07H 1/00, C07H 11/00, A61K 9/127, A61P 35/00

(54) **PROCESS FOR THE PREPARATION OF SUCROSE OCTASULFATE OCTAKISTRIETHYLAMMONIUM SALT (TASOS) POWDER AND USES THEREOF**

(71) Applicant: SANDOZ AG, 4056 Basel (CH)
(72) Inventor: ZUPANCIC, Borut, 1526 Ljubljana (SI)
(74) Representative: Prüfer & Partner mbB Patentanwälte · Rechtsanwälte

(57) **Abstract**

The present invention refers to a process for preparing sucrose octasulfate octakistriethylammonium salt (TASOS) powder, comprising the steps of combining a TASOS solution with a polar organic solvent(s) and triethylamine, thereby obtaining a TASOS solution, the polar organic solvent(s), and triethylamine; concentrating said TASOS solution; combining it with 2-methyltetrahydrofuran and/or tetrahydrofuran and triethylamine, thereby obtaining a mixture; and stirring said mixture until a white TASOS powder is obtained.

The present invention also refers to a sucrose octasulfate octakistriethylammonium salt (TASOS) powder having a purity of at least 96%, the use of the sucrose octasulfate octakistriethylammonium salt (TASOS) powder for preparing a pharmaceutical composition, and the use of tetrahydrofuran or 2-methyltetrahydrofuran as antisolvent for the preparation of TASOS powder.

Finally, the present invention refers to a pharmaceutical composition prepared by using the sucrose octasulfate octakistriethylammonium salt (TASOS) powder of the present invention, and to the pharmaceutical composition for use in treating cancer.

## Description

### Field of the invention

The present invention refers to a process for preparing sucrose octasulfate octakistriethylammonium salt (TASOS) powder, comprising the steps of combining a TASOS solution with a polar organic solvent(s) and triethylamine, thereby obtaining a TASOS solution, the polar organic solvent(s), and triethylamine; concentrating said TASOS solution; combining it with 2-methyltetrahydrofuran and/or tetrahydrofuran and triethylamine, thereby obtaining a mixture; and stirring said mixture until a white TASOS powder is obtained.

The present invention also refers to a sucrose octasulfate octakistriethylammonium salt (TASOS) powder having a purity of at least 96%, the use of the sucrose octasulfate octakistriethylammonium salt (TASOS) powder for preparing a pharmaceutical composition, and the use of tetrahydrofuran or 2-methyltetrahydrofuran as antisolvent for the preparation of TASOS powder.

Finally, the present invention refers to a pharmaceutical composition prepared by using the sucrose octasulfate octakistriethylammonium salt (TASOS) powder of the present invention, and to the pharmaceutical composition for use in treating cancer.

### Description of the background art

Sucrose octasulfuric acid is an ester of sucrose wherein all hydroxy groups are esterified by sulfuric acid. The acidic form itself is not stable and rapidly hydrolyses to less substituted compounds. Salts like sodium, potassium and ammonium salts are more stable and can be isolated as crystalline compounds with long time stability. An aluminum complex with sucrose octasulfuric acid known as sucralfate is used as an agent for treatment of gastroesophageal reflux disease.

Recently, sucrose octasulfate salt of irinotecan was developed for treatment of different cancers. Irinotecan (7-ethyl-10-[4-(1-piperidino)-1-piperidino]carbonyloxycampothecin) is a water-soluble camptothecin derivative used in cancer chemotherapy. The pharmacology of irinotecan is complex, and in order to improve its pharmacokinetic and also its biodistribution, it is desirable to provide a stable pharmaceutical packaging technology. It has been found that encapsulating irinotecan into liposome-based nanoparticles (liposomal vesicles/liposomal products) provides for the desired stability. Irinotecan sucrose octasulfate in a pharmaceutical product that was developed for treating different cancers is a liposomal product, wherein all irinotecan sucrose octasulfate is packed inside of a liposomal vesicle. The accumulation of the salt in the liposomal vesicle is prepared by a salt of sucrose octasulfuric acid with an anion, which easily passes through the vesicle membrane. Triethylammonium salt was recognized as the most suitable salt.

Until now, sucrose octasulfate octakistriethylammonium salt (TASOS) was prepared from sodium or potassium salt by ion exchange in a column. The resulting aqueous TASOS solution was to be partially evaporated in order to adjust the desired volumes and the desired concentration. However, the manipulation in water results in a partial degradation of the ester, thereby forming less substituted sulfates, finally resulting in unwanted degradation products, in particular in heptasulfate impurities. An eventual storage of the TASOS solution comprising water can further contribute to enhancing the concentration of impurities, which can challenge the preparation of products comprising sucrose octasulfate, particularly the preparation of liposomal product.

Furthermore, in order to shorten the manipulation times (to reduce the risk of contaminating the product) this chemical process of preparing TASOS solution had to be transferred to the pharmaceutical manufacturing plant, wherein the sterility is an extremely challenging condition. A solid stable form of TASOS is a solution for these challenges.

In this regard, US4990610 discloses the preparation of solid sucrose octasulfate ammonium salt from an aqueous mixture. The procedure as described is not applicable for TASOS powder preparation, because aqueous conditions are present, which do not allow solidification of TASOS.

Ochi et al. (Chem. Pharm. Bull. 1980, 28, 638) describe the preparation of crystalline sucrose octasulfate salts. The salts that are used are sodium, potassium, barium, rubidium, cesium and ammonium. Crystalline sucrose octasulfate salts are prepared from aqueous methanol. The procedure as described is not applicable for TASOS powder preparation, because aqueous conditions are present, which do not allow solidification of TASOS

US10456360, US2017319573, US2018296484, US2020069586 and WO2017066726 respectively disclose the preparation of sucrose octasulfate triethylammonium salt and sucrose octasulfate diethylammonium aqueous solutions. Again, the procedure as described is not applicable for TASOS powder preparation, because aqueous conditions are present, which do not allow solidification of TASOS.

Yang et al. (Biomater. Sci. 2019, 7, 419) describe the preparation of sucrose octasulfate triethylammonium salt aqueous solution similar to US10456360.

Drummond et al. (Cancer Research 2006, 66, 3271) describes the preparation of sucrose octasulfate triethylammonium salt aqueous solution similar to US10456360.

However, prior art processes intented to prepare usable particles from aqueous or alcoholic solutions, but completely failed due to resulting gel-like or gum-like solid products. Hence, there is still a need for a process for preparing TASOS powder which is suitable for being used in pharmaceutical compositions, as well as for such an improved TASOS powder itself, wherein this TASOS powder is improved e.g. with regard to purity and/or stability, e.g. with regard to a reduction of sulfated impurities. It is thus an object of the present invention to provide an industrially applicable and economically acceptable process for the preparation of sucrose octasulfate octakistriethylammonium salt (TASOS) powder, starting from TASOS solution.

### Summary of the invention

According to the prior art preparation procedures described above, TASOS is prepared from solutions. TASOS by its nature has some drawbacks, for example it is very hygroscopic and thus, even if only traces of water are present, gummy material is obtained. But even if no water is contained in the solutions TASOS is prepared from, due to the lack of stability, prior art preparation processes result in solid TASOS that exhibits a comparably high level of impurities. Hence, the prior art processes do not allow formation of solid TASOS in pharmaceutical grade.

Therefore, TASOS obtained by procedures described in the prior art is isolated as solution (e.g. aqueous solution) or as solid containing impurities; this solid TASOS is thus not of pharmaceutical grade and has for example a purity of (only) 90 area %.

The solution to resolve all these challenges is a solid stable form of TASOS. A solid material is also easier to handle and consequently more precise dosing in the final dosage form (FDF) process is possible. The present invention is based on the unexpected finding that such a solid stable form of TASOS can be provided by using the process described herein.

It has additionally been found that, if a commercial solid TASOS, which is not qualified for being used in pharmaceutical production, is subjected to the process of the present invention, the resulting TASOS powder e.g. has a purity of at least 96 area-%, as determined by Ultra Performance Liquid Chromatography (UPLC). Hence, the resulting TASOS powder is suitable for being used in pharmaceutical products. Most notably, when TASOS fine powder is isolated according to the present invention, the main heptasulfate impurities are significantly reduced: HS1 (heptasulfate impurity) is reduced by about 55 - 60%, HS2 is totally removed, and HS3 is reduced by about 45 - 50%. The impurities were identified based on MS analysis.

### Detailed description of the invention

The invention is based on solidification of TASOS in a water free environment. In a first step of the process of the present invention, water is removed by evaporation, or solvent exchange is carried out, thereby arriving at a TASOS solution. The solvent that is present in this TASOS solution is more polar than the solvent that is used as antisolvent in the second step. In the second step, the solvent that has been used in the first step is step-wise and slowly exchanged by the less polar solvent tetrahydrofuran or 2-methyltetrahydrofuran, thereby resulting in a precipitation of TASOS as powder, preferably white powder. Repeating the respective solvent exchange steps, which comprise a concentration step and a solvent re-addition step, at least once, contributes to obtaining TASOS as a white powder exhibiting pharmaceutical grade purity, as well as ensuring a pH value above 7, preferably above 9.

One key factor for obtaining solid TASOS as a powder, if water is present in the starting material, is efficient water removal, which is ensured by azeotropic removal during solvent exchange to acetonitrile or other polar organic solvents. In case traces of water are still present in the solution, they are further removed during solvent exchange to 2-methyltetrahydrofuran and/or tetrahydrofuran, which is also an antisolvent (or antisolvent mixture) responsible for precipitation of TASOS. Another important factor during solvent exchanges is the addition of triethylamine to ensure pH above 7, preferably above 9, which prevents degradation of TASOS during the solidification process.

In the process instead of e.g., acetonitrile also other polar organic solvents that can form azeotrope with water can be used (e.g. ethanol) and solvents, which do not form azeotrope with water (e.g. methanol). If a solvent that does not form an azeotrope with water is used, water needs to be efficiently removed in the second step, where solvent is exchanged with the antisolvent(s) 2-methyltetrahydrofuran and/or tetrahydrofuran.

The present invention thus refers to a process for preparing sucrose octasulfate octakistriethylammonium salt (TASOS) powder from a TASOS solution as defined in the claims.

Starting from a TASOS solution from which TASOS powder cannot be obtained, the aim of the process is to provide a TASOS solution from which TASOS powder can be precipitated. It was unexpectedly found how a TASOS powder can be precipitated when starting from a TASOS solution, particularly from a TASOS solution comprising water.

Since water hampers the precipitation of TASOS, in the process of the present invention water is not added on purpose, but can be present as impurity, e.g., from the process of preparing TASOS itself. In this case, if water is present in the TASOS solution, it has to be removed in the inventive process. In this case, water is replaced with the polar organic solvent(s) such as acetonitrile. Particularly, water has to be removed to a sufficient degree, i.e. low water concentration, in order that the addition of the antisolvent(s) 2-methyltetrahydrofuran and/or tetrahydrofuran finally results in precipitation of TASOS powder. The concentration of water during the precipitation step should be as low as possible to allow precipitation. It is preferred that during the precipitation step, water is removed completely.

If no water is present as impurity, the process of the invention also allows providing a TASOS solution from which TASOS powder can be precipitated. In this case, the TASOS solution in a solvent comprising one or more polar organic solvents can be concentrated to result either in a slurry or in a TASOS solution, preferably a clear TASOS solution. To the slurry, polar solvent can be added to provide a TASOS solution, preferably a clear TASOS solution. The resulting TASOS solutions are preferably more concentrated than the TASOS solution in step a). This concentration step is part of the solvent exchange to methyltetrahydrofuran and/or tetrahydrofuran which allows for the solvent exchange with 2-methyltetrahydrofuran and/or tetrahydrofuran from which TASOS powder is eventually precipitated.

The structure of sucrose octasulfate octakistriethylammonium salt (TASOS) is as follows:

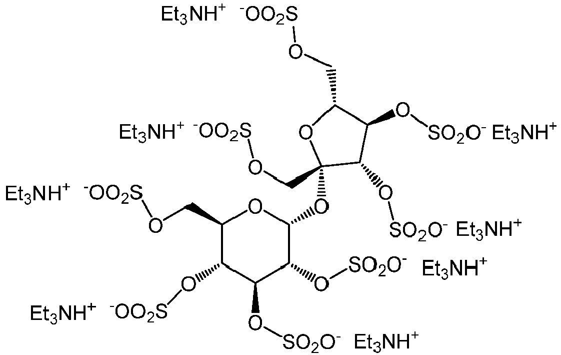

According to step a) of the process of the invention, TASOS solution comprising a polar organic solvent or a mixture of polar organic solvents, triethylamine, and optionally water, is provided. At least one of the polar organic solvent, or mixture of polar organic solvents, has a polarity that is greater than the polarity of 2-methyltetrahydrofuran or tetrahydrofuran, depending on which solvent is used as antisolvent later in the inventive process. In a preferred embodiment, if 2-methyltetrahydrofuran is used as antisolvent, the at least one of the polar organic solvent or mixture of polar organic solvents has a polarity that is greater than 2-methyltetrahydrofuran, and if tetrahydrofuran is used as antisolvent, at least one of the polar organic solvent or mixture of polar organic solvents has a polarity that is greater than tetrahydrofuran.

Triethylamine is present and ensures a pH value above 7, preferably a pH value of 9 or higher, and that TASOS is stably present as the octakistriethylammonium salt. The amount of triethylamine used is preferably up to 5%, preferably 2%. The amount of triethylamine used in step a) and e) and when repeating steps a) and e) can be the same or different, and is respectively up to about 5 wt.-%, preferably about 2 wt.-%, based on the amount of organic solvent or mixture of organic solvents.

The TASOS solution of step a) can be obtained from sucrose octasulfonic acid and salts thereof, e.g. ammonium salt thereof (sucrose octasulfate triethylammonium salt), sodium salt thereof (sodium sucrose octasulfate), or potassium salt thereof (potassium sucrose octasulfate (KSOS)).

When the TASOS solution is obtained from sucrose octasulfonic acid and salts thereof, other than from sucrose octakistriethylammonium salt (TASOS), a cation exchange step has to be carried out before step a) of claim 1. Example 4 describes a cation exchange to get TASOS from KSOS. Furthermore, also prior art TASOS solids that are not of pharmaceutical grade can be used and dissolved in one or more polar organic solvents as defined in the claims, for example at room temperature, e.g., 20-25°C. If the TASOS to be used is already in the form of a solution, one or more additional polar organic solvents as defined in the claims can be added. It is also possible to firstly remove the solvents from the TASOS solution and then add one or more polar organic solvents as defined in the claims.

If water is present in the TASOS solution of step a), in the next steps b) and c), a solvent exchange step is carried out to remove the water. After the solvent exchange, TASOS solution which is essentially free of water is obtained. The maximum amount of water in the water-free organic solvent is preferably 0.5% by weight of the TASOS solution, in particular 0.5% when using acetonitrile as the polar organic solvent in step a) and 0.2% when using 2-methyltetrahydrofuran and/or tetrahydrofuran.

The amount of polar organic solvent or mixture of polar organic solvents in step a) is chosen to maintain a TASOS solution without formation of precipitates, for example, the TASOS solution can be 100g TASOS/200 mL acetonitrile.

The polar organic solvent of step b) can be a solvent which forms an azeotrope with water or can be a solvent which does not form an azeotrope with water. In the mixture of polar organic solvents in step b), each of the solvents can either be a solvent which forms an azeotrope with water or can be a solvent which does not form an azeotrope with water. In a preferred embodiment of the present invention, the polar organic solvent is a solvent which forms an azeotrope with water. In another preferred embodiment the first mixture of organic solvents contains at least a solvent which forms an azeotrope with water, further preferred, the mixture only contains solvents which form an azeotrope with water.

The polarity of the organic solvents of step b) is higher than the polarity of 2-methyltetrahydrofuran and/or tetrahydrofuran. Preferably, the polarity of the solvents is defined based on the dipole moment (D) at 25°C which can be determined from the Debye equation based on the dielectric constant. Dielectric constants for solvents are, for example, listed in the "Dielektrizitatskonstante (DK-Wert) Kompendium", version available on 22 October 2021, at https://docplayer.org/53720533-Dielektrizitaetskonstante-dk-wert-kompendium.html. The dipole moment/dielectric constants for 2-methyltetrahydrofuran and/or tetrahydrofuran and the polar organic solvents are measured/determined by using the same method in order that the values are comparable.

If water is present in step a), steps a) and b) have to be repeated one or more times using the same or a different polar organic solvent or the same or a different mixture of polar organic solvents in order to efficiently remove water. This is referred to herein as the "first solvent exchange step". It is not necessary to measure the water content after each repetition. Rather, it has to be determined at the beginning of the process whether water is present in the starting material. The process has then to be adapted in order that the degree of concentration and number of repetitions of steps a) and b) provide a sufficiently low water concentration that the TASOS powder can finally be achieved. Since water can also be removed when performing the solvent exchange to 2-methyltetrahydrofuran and/or tetrahydrofuran (this is referred to herein as the "second solvent exchange step"), the water does not have to be removed to the final water content in the first solvent exchange step. For example, if water is present in the starting solution of step a), this water does not have to be removed completely in the first solvent exchange step, but remaining water can be removed in the second solvent exchange step.

If no water is present, steps a) and b) do not need to be repeated. Of course it is also possible to repeat said steps.
If no water is present in the TASOS solution provided in step a), it is not mandatory (but possible) to carry out concentration step b). Hence, concentrating the TASOS solution of step a) is optional. It is possible to carry out a concentration step b), and it is also possible to not carry out a concentration step b). If no concentration step is carried out, the TASOS solution of step a) is directly combined with the antisolvent(s) 2-methyltetrahydrofuran and/or tetrahydrofuran. In this case, after step a), step e) is carried out. If a concentration step is carried out, then the concentrated solution of step b) is combined with the antisolvent(s) 2-methyltetrahydrofuran and/or tetrahydrofuran. In this case, after step b), step e) is carried out. It is however also possible to carry out step d) prior to carrying out step e).

In step d) a further amount of the polar organic solvent(s) from step a) is added to the slurry, thereby obtaining a, preferably clear, TASOS solution.

If the TASOS solution does not contain water but is merely concentrated in step b), it may not be necessary to add the further amount of polar organic solvent(s) of step d).

In step e), the TASOS solution, preferably clear TASOS solution, of step a) (when no water is present in the solution provided in step a)), of step b), or of step d) is combined with 2-methyltetrahydrofuran and/or tetrahydrofuran (second solvent exchange to less polar organic solvent); and triethylamine in order to ensure a pH value above 7, preferably above 9, thereby obtaining a mixture. Due to the presence of the antisolvent (2-methyltetrahydrofuran and/or tetrahydrofuran), TASOS could already start to precipitate and the mixture comprises particles (precipitate). Particularly, if the TASOS solution is combined with the antisolvent 2-methyltetrahydrofuran, precipitation of TASOS can already start, thereby forming a mixture.

In step e), the organic solvent (2-methyltetrahydrofuran and/or tetrahydrofuran), and triethylamine, are combined with the preferably clear TASOS solution over a time period of 0.5 to 2 hours, preferably 0.75 and 1.5 hours, more preferably about 1 hour, at a temperature in the range of from about 10°C to about 40°C, preferably in the range of about 20°C to 30°C, and more preferably at about 25°C. Upon combining the TASOS solution with the organic solvent, in particular with 2-methyltetrahydrofuran, a precipitate can form.

In step f) the TASOS combination of step e) is again concentrated to obtain a slurry.

In step g), the steps e) and f) are repeated one or more times. This is crucial for achieving the required solvent exchange and to eventually precipitate the desired TASOS powder. According to the invention, it is preferred that at least steps a/b or e/f are repeated one or more times. Further preferred both steps a) and b) and e) and f) are repeated one or more time.

Then, in step h), a further amount of 2-methyltetrahydrofuran and/or tetrahydrofuran is added to the concentrated TASOS combination, thereby obtaining a mixture.

The obtained TASOS mixture can be stirred over a time period of 0.5 to 2 hours, preferably 0.75 and 1.5 hours, more preferably about 1 hour, at a temperature in the range of from about 10°C to about 75°C, preferably in the range of about 20°C to 50°C, more preferably in the range of about 20°C to about 40°C, most preferred at about 25°C.

In steps e) and/or h), 2-methyltetrahydrofuran and/or tetrahydrofuran is added over a time period of 0.5 to 2 hours, preferably 0.75 and 1.5 hours, more preferably about 1 hour, at a temperature in the range of from about 10°C to about 75°C, preferably in the range of about 20°C to 50°C, more preferably in the range of about 20°C to about 40°C, most preferred at about 25°C;
preferably, in steps e) and h), 2-methyltetrahydrofuran and/or tetrahydrofuran is added over a time period of 0.5 to 2 hours, preferably 0.75 and 1.5 hours, more preferably about 1 hour, at a temperature in the range of from about 10°C to about 75°C, preferably in the range of about 20°C to 50°C, more preferably in the range of about 20°C to about 40°C, most preferred at about 25°C.

In step i) the obtained mixture is stirred until a TASOS powder is obtained, i.e. precipitated. The stirring can be carried out over a time period of 10 to 25 hours, preferably 12 to 20 hours, more preferably 14 to 18 hours, and most preferred at about 16 hours, at a temperature in the range of from about 10°C to about 40°C, preferably in the range of about 20°C to 30°C, and more preferably at about 25°C. TASOS solid is obtained as filterable fine powder.
In a preferred embodiment, this TASOS powder is white, which is indicative for the beneficial purity of the obtained TASOS powder.

In step j) the TASOS powder of step i) can optionally be filtered off and washed.

In step k) the TASOS powder can optionally be dried.

The TASOS powder obtained is in amorphous form.

In the following, the solvents used for the first solvent exchange are described in more detail:
If water is present in the TASOS solution of step a), one or more, preferably all, organic solvent(s) in step a) form an azeotrope with water.

In order that water (if present) is removed in the concentration step b) when a non-azeotropic organic solvent is used, the boiling point of the organic solvent generally has to be higher than the boiling point of water.

The polar organic solvent or the mixture of polar organic solvents in step a) is preferably selected from the group consisting of acetonitrile, ethanol, and methanol, preferably the polar organic solvent is acetonitrile or ethanol, more preferably the polar organic solvent is acetonitrile.
The polar organic solvent in step e) is 2-methyltetrahydrofuran and/or tetrahydrofuran, preferably 2-methyltetrahydrofuran.

In one embodiment, the solvent of step a) and step d) is the same solvent. Preferably, the solvent of step d) is acetonitrile, further preferred the solvent of step a) and of step d) is acetonitrile.

The concentration step b) and/or the concentration step f) is preferably carried out at reduced pressure and at a temperature in the range of from about 10°C to about 75°C, preferably in the range of about 20°C to 50°C, more preferably in the range of about 20°C to about 40°C, most preferred at about 25°C; preferably, the concentration step b) and the concentration step f) are carried out at reduced pressure and at a temperature in the range of from about 10°C to about 75°C, preferably in the range of about 20°C to 50°C, more preferably in the range of about 20°C to about 40°C, most preferred at about 25°C.
The solvent exchange to acetonitrile is preferably performed at temperature 10°C - 40°C, preferably at the range of 20°C - 30°C, most preferably at 25°C. The evaporation is preferably performed in a slow manner.

In the concentration step b) and/or the concentration step f) between 45% and 95% of the solvent(s), preferably between 50% and 90%, more preferably between 60% and 80%, most preferred about 75%, of the solvent(s) is preferably removed;
Further preferred, in the concentration step b) and in the concentration step f) between 45% and 95% of the solvent(s), preferably between 50% and 90%, more preferably between 60% and 80%, most preferred about 75%, of the solvent(s) is removed.

The concentration step b) and/or the concentration step f) can be an evaporation step.

The unexpected finding of the present invention is that repeating steps a/b and/or e/f provides a particularly suitable TASOS solution for the precipitation of a TASOS amorphous powder.
It is not common to purify an amorphous compound via precipitation. The reason is that for an amorphous powder, it cannot reliably be predicted whether the precipitation will lead to a product with increased purity. Contrary thereto, purifying by means of crystallization is expected to result in products exhibiting increased purity. Thus, in the present invention, it has surprisingly been found that the claimed process results in TASOS powder exhibiting pharmaceutical grade, although the TASOS obtained is in amorphous form.

In a preferred embodiment, in the process,
- if water is present, steps a) and b), and steps e) and f), or
- if no water is present in the TASOS solution provided in step a), only steps e) and f),
are repeated 1 to 20 times, preferably 2 to 15 times, more preferably 3 to 10 times, and most preferably 5 times.

In the second solvent exchange step of the process according to the present invention, starting at step e), TASOS is purified during solidification process and filtration of the thus-obtained solid product. During TASOS solution synthesis less substituted sulfate impurities, mainly heptasulfate impurities are formed. Eight possible positional isomers can be formed three main of them are identified as chromatographic peaks, defined by area %: heptasulfate impurity 1 (HS1), heptasulfate impurity 2 (HS2) and heptasulfate impurity 3 (HS3). Amounts of all three main impurities are reduced during the process of TASOS amorphous powder preparation. Typically HS1 is reduced by 55 - 60%, HS2 is totally removed, and HS3 is reduced by 45 - 50%.

The invention thus also refers to sucrose octasulfate octakistriethylammonium salt (TASOS) powder having a purity of at least 96 area %, preferably at least 97 area %, more preferably of at least 98 area %, as determined by Ultra Performance Liquid Chromatography (UPLC).

The TASOS powder can have a particle sized distribution (PSD) d90 of 50-120 µm and/or a d10 of 3-10 µm and/or a d50 of 15-60 µm. The PSD can e.g., be determined by using a volumetric dynamic laser light scattering method, preferably by using an instrument of Malvern Instruments, Ltd., Malvern, UK, preferably a Malvern Mastersizer 3000.

The TASOS powder can have a residual solvent content of 2-methyltetrahydrofuran or tetrahydrofuran, preferably of less than 1% or 4000 ppm as determined by gas chromatography (GC).

The TASOS powder can have a specific BET surface area of at least 0.20 m²/g. BET surface area measurements can e.g., be performed in accordance with ISO 9277:2010(E) standard with nitrogen and e.g., static volumetric and multipoint methods.

The TASOS powder can have a tapped density of less than 1.0, preferably less than 0.80, more preferably less than 0.70, and most preferably less than 0.60 g/mL.

The invention also refers to the use of the sucrose octasulfate octakistriethylammonium salt (TASOS) powder as disclosed herein for preparing a pharmaceutical composition.

The sucrose octasulfate octakistriethylammonium salt (TASOS) powder as disclosed herein can be used for preparing a pharmaceutical composition, preferably a liposomal composition. For example, the TASOS powder of the present invention can be used for preparing a composition.

The invention also refers to the use of 2-methyltetrahydrofuran and/or tetrahydrofuran as antisolvent for the preparation of TASOS powder.

The invention also refers to a pharmaceutical composition prepared by using the sucrose octasulfate octakistriethylammonium salt (TASOS) powder as disclosed herein. The pharmaceutical composition can comprise liposomes and entrapped inside the liposomes irinotecan with sucrose octasulfate (SOS) as drug entrapment agent.

The present invention also refers to TASOS powder obtained by applying a process as defined herein.

Finally, the present invention refers to the pharmaceutical composition disclosed herein for use in treating cancer, preferably pancreatic cancer, colon cancer and/or small cell lung cancer.

### Examples

### Example 1: Preparation of TASOS solution in acetonitrile

To a TASOS (100 g) aqueous solution (1 L) acetonitrile (with 2% triethylamine, 750 mL) was added and the obtained mixture was concentrated at 25°C. To the obtained slurry acetonitrile (with 2% triethylamine, 750 mL) was added and concentrated at 25°C. This step was repeated five times. After the last concentration step, acetonitrile (with 2% triethylamine, 200 mL) was added to obtain TASOS solution.

### Example 2: Preparation of TASOS white powder from acetonitrile solution

All the operations were performed under nitrogen atmosphere: To a TASOS (100 g) acetonitrile (200 mL) was added. To the obtained clear solution 2-methyltetrahydrofuran (with 2% triethylamine, 750 mL) was added at 25°C in 1 h. Obtained mixture was stirred at 25°C for 1 h and then ∼75% of the solvent was evaporated at 25°C. To the mixture 2-methyltetrahydrofuran (with 2% triethylamine, 750 mL) was added at 25°C in 1 h. Obtained mixture was stirred at 25°C for 1 h and then ∼75% of the solvent was evaporated at 25°C. This step was repeated five times. After last evaporation 2-methyltetrahydrofuran (with 2% triethylamine, 750 mL) was added at 25°C in 1 h and obtained mixture was stirred at 25°C for 16 h. Obtained white powder was filtered, washed with 2-methyltetrahydrofuran (with 2% triethylamine, 250 mL) and dried in vacuum at 25°C to afford TASOS as white amorphous powder (92.4 g, 92%). UPLC analysis: Sucrose octasulfate 98.14 area%, HS1 0.99 area%, HS2 <DL, HS3 0.34 area%, sulfate 0.52 area%.
UPLC analysis of starting TASOS: Sucrose octasulfate 97.04 area%, HS1 1.25 area%, HS2 0.39 area%, HS3 0.63 area%, sulfate 0.68 area%.
PSD analysis (Malvern): d10 5.6 µm, d50 30.1 µm, d90 78.8 µm
Residual solvents: MeTHF 2291 ppm
SSA: 0.29 m²/g
Tapped density: 0.51 g/mL

### Example 3: Preparation of potassium sucrose octasulfate (KSOS)

A reactor (6 L) was loaded with anhydrous pyridine (1.90 L) at 20°C followed by pyridine sulfur trioxide (619 g, 3.89 mol). A white suspension formed with no significant temperature change. The mixture was heated to 45°C and sucrose (164 g, 0.48 mol) was added. After 15 min the reaction temperature increased to 80°C. A resinous product separated. The mixture was stirred for 6 h. Stirring was stopped, the temperature was reduced to 20°C and most of the supernatant (1.20 kg) was removed by suction trough an immersed pipe. The solidified product was dissolved in water (2.0 L) at 20°C. When all dissolved, a solution of 10% KOH (aq.) was slowly added up to pH 9.0 (∼2.5 L). Methanol (1.5 L) was added in 30 min. A suspension was formed. After additional 15 min of stirring, the precipitate was isolated by vacuum filtration, the reactor and filtering cake was washed with methanol/water (1:1, 2 × 1 L), methanol (0.5 L), and dried at 50°C overnight under reduced pressure to get the KSOS crude (613 g).
A reactor (20 L) was loaded with KSOS crude (613 g) and water (5.5 L). The suspension stirred at 25°C to dissolve into a clear solution. Methanol (5.5 L) was added during 3 h at constant flow at 25°C. The obtained suspension was stirred for 1 h, the product isolated by filtration, and the filter cake washed with methanol (1 L). The product was dried at 50°C under reduced pressure to give a white powder (557 g, 91% overall yield; 99.85 area% purity by HPLC).

### Example 4: Preparation of TASOS from KSOS

KSOS (100 g, 78 mmol; S-LIP-1-313-03) was diluted with water to 1 L and the suspension stirred at room temperature till dissolution is achieved. A small amount of insolubles is removed by filtration. This solution is then subjected to flow through a regenerated Dowex column on Biotage Isolera using a 1 : 1 dilution with water (50 g/L isocratic) at a flow rate of 50 mL/min (on consumption of KSOS, its flask and the system was washed with 100 mL water). The first 100 mL is discarded. Fractions were collected in 100 mL flask containing 6 mL of triethylamine. Fractions were combined and evaporated under reduced pressure (∼10 mbar) at 30°C with addition of 10 mL triethylamine each 30 min to obtain viscous colorless oil (pH 8.67, HPLC analysis 98.36 area% SOS).
To the viscous oil acetonitrile (with 2% triethylamine, 750 mL) was added and obtained solution was concentrated at 25°C. To the obtained slurry acetonitrile (with 2% triethylamine, 750 mL) was added and concentrated at 25°C. This step was repeated five times to afford TASOS, ready for the TASOS solidification steps of the process as claimed and exemplified in Example 2.

## Claims

1. Process for preparing sucrose octasulfate octakistriethylammonium salt (TASOS) powder, comprising the steps of:
a) providing a TASOS solution comprising
a polar organic solvent, or a mixture of polar organic solvents, wherein at least one of the polar organic solvents, has a greater polarity than 2-methyltetrahydrofuran or tetrahydrofuran;
triethylamine in order to ensure a pH value above 7; and
optionally water;
b) concentrating the TASOS solution of step a) by removing water if present, and optionally said polar organic solvent or mixture of polar organic solvents; or, if no water is present, optionally concentrating the TASOS solution of step a) by removing said polar organic solvent or mixture of said polar organic solvents; thereby obtaining a slurry or a solution;
c) if water is present in step a), repeating steps a) and b) one or more times using the same or a different polar organic solvent or the same or a different mixture of polar organic solvents;
d) adding a further amount of the polar organic solvent(s) from step a) to the slurry, thereby obtaining a TASOS solution;
e) combining the TASOS solution of step a), of step b), or of step d) with 2-methyltetrahydrofuran and/or tetrahydrofuran; and
triethylamine in order to ensure a pH value above 7;
thereby obtaining a mixture;
f) concentrating the TASOS mixture of step e);
g) repeating steps e) and f) one or more times;
h) adding a further amount of 2-methyltetrahydrofuran and/or tetrahydrofuran, thereby obtaining a mixture;
i) stirring the obtained mixture until a TASOS powder is obtained;
j) optionally filtering and washing the TASOS powder of step i);
k) optionally drying the TASOS powder.

2. Process according to claim 1, wherein, if water is present in the TASOS solution of step a), one or more, preferably all, organic solvent(s) in step a) form an azeotrope with water.

3. Process according to any of the preceding claims, wherein the polar organic solvent or the mixture of polar organic solvents in step a) is selected from the group consisting of acetonitrile, ethanol, and methanol, and/or the polar organic solvent in step e) is 2-methyltetrahydrofuran and/or tetrahydrofuran.

4. Process according to any of the preceding claims, wherein the concentration step b) and/or the concentration step f) is carried out at reduced pressure and at a temperature in the range of from about 10°C to about 75°C, preferably in the range of about 20°C to 50°C, more preferably in the range of about 20°C to about 40°C, most preferred at about 25°C.

5. Process according to any of the preceding claims, wherein in the concentration step b) and/or the concentration step f) between 45% and 95% of the solvent(s), preferably between 50% and 90%, more preferably between 60% and 80%, most preferred about 75%, of the solvent(s) is removed;
preferably, in the concentration step b) and in the concentration step f) between 45% and 95% of the solvent(s), preferably between 50% and 90%, more preferably between 60% and 80%, most preferred about 75%, of the solvent(s) is removed.

6. Process according to any of the preceding claims, wherein
- if water is present, steps a) and b), and steps e) and f), or
- if no water is present in step a), only steps e) and f),
are repeated 1 to 20 times, preferably 2 to 15 times, more preferably 3 to 10 times, and most preferably 5 times.

7. Process according to any of the preceding claims, wherein in step e), the organic solvent, and triethylamine, are combined with the TASOS solution over a time period of 0.5 to 2 hours, preferably 0.75 and 1.5 hours, more preferably about 1 hour, at a temperature in the range of from about 10°C to about 40°C, preferably in the range of about 20°C to about 30°C, and more preferably at about 25°C.

8. Process according to any of the preceding claims, wherein in step e), the obtained TASOS solution is stirred over a time period of 0.5 to 2 hours, preferably 0.75 and 1.5 hours, more preferably about 1 hour, at a temperature in the range of from about 10°C to about 75°C, preferably in the range of about 20°C to about 50°C, more preferably in the range of about 20°C to about 40°C, most preferred at about 25°C.

9. Process according to any of the preceding claims, wherein in steps e) and/or h), 2-methyltetrahydrofuran and/or tetrahydrofuran is added over a time period of 0.5 to 2 hours, preferably 0.75 and 1.5 hours, more preferably about 1 hour, at a temperature in the range of from about 10°C to about 75°C, preferably in the range of about 20°C to about 50°C, more preferably in the range of about 20°C to about 40°C, most preferred at about 25°C;
preferably, in steps e) and h), 2-methyltetrahydrofuran and/or tetrahydrofuran is added over a time period of 0.5 to 2 hours, preferably 0.75 and 1.5 hours, more preferably about 1 hour, at a temperature in the range of from about 10°C to about 75°C, preferably in the range of about 20°C to about 50°C, more preferably in the range of about 20°C to about 40°C, most preferred at about 25°C;
and/or
wherein in step i), the stirring is carried out over a time period of 10 to 25 hours, preferably 12 to 20 hours, more preferably 14 to 18 hours, and most preferred at about 16 hours, at a temperature in the range of from about 10°C to about 40°C, preferably in the range of about 20°C to about 30°C, and more preferably at about 25°C.

10. Sucrose octasulfate octakistriethylammonium salt (TASOS) powder having a purity of at least 96 area %, preferably at least 97 area %, more preferably of at least 98 area %, as determined by Ultra Performance Liquid Chromatography (UPLC).

11. The TASOS powder of claim 10, having
i) a particle sized distribution (PSD) d90 of 50-120 µm and/or a d10 of 3-10 µm and/or a d50 of 15-60 µm, as determined by Malvern Mastersizer; and/or
ii) a residual solvent content of 2-methyltetrahydrofuran or tetrahydrofuran, preferably of less than 1% or 4000 ppm as determined by gas chromatography (GC); and/or
iii) a specific BET surface area of at least 0.20 m²/g; and/or
iv) a tapped density of less than 1.0, preferably less than 0.80, more preferably less than 0.70, and most preferably less than 0.60 g/mL.

12. Use of the sucrose octasulfate octakistriethylammonium salt (TASOS) powder as defined in claim 10 or 11 for preparing a pharmaceutical composition.

13. Use of 2-methyltetrahydrofuran or tetrahydrofuran as antisolvent for the preparation of TASOS powder.

14. A pharmaceutical composition prepared by using the sucrose octasulfate octakistriethylammonium salt (TASOS) powder as defined in claim 10 or 11.

15. The pharmaceutical composition of claim 14 for use in treating cancer, preferably pancreatic cancer, colon cancer, and/or small cell lung cancer.
